# EUROPEAN PATENT SPECIFICATION

(11) **EP 1 799 638 B1**
(45) Date of publication and mention of the grant of the patent: **30.07.2008**
(21) Application number: 05794715.2
(22) Date of filing: 19.09.2005
(51) Int. Cl.: C07C 319/20, C07C 323/22

(54) **PROCESS FOR PREPARING AROMATIC THIOPHENYL KETONES**
VERFAHREN ZUR HERSTELLUNG AROMATISCHER THIOPHENYLKETONE
PROCÉDÉ DE PREPARATION DE CÉTONES AROMATIQUES THIOPHÉNYL

(30) Priority: 29.09.2004 EP 04104742
(43) Date of publication of application: 27.06.2007
(73) Proprietor: Ciba Holding Inc., 4057 Basel (CH)
(72) Inventor: PRÉTÔT, Roger, CH-4054 Basel (CH); VAN DER SCHAAF, Paul Adriaan, F-68220 Hagenthal-le-Haut (FR); KUGLER, Rolf, 79591 Eimeldingen (DE); WOLF, Walter, 79713 Bad Säckingen (DE)
(74) Representative: Hoechst, Bruno Werner
(86) International application number: PCT/EP2005/054638
(87) International publication number: WO 2006/034966

(56) References cited:
- EP-A- 0 003 002
- WO-A-02/42254
- US-A- 5 883 267
- YADAV G D ET AL: "Novelties of synthesis of acetoveratrone using heteropoly acid supported on hexagonal mesoporous silica" MICROPOROUS AND MESOPOROUS MATERIALS, ELSEVIER SCIENCE PUBLISHING, NEW YORK, US, vol. 63, no. 1-3, 18 September 2003 (2003-09-18), pages 85-96, XP004455491 ISSN: 1387-1811
- KOZHEVNIKOV I V: "Friedel-Crafts acylation and related reactions catalysed by heteropoly acids" APPLIED CATALYSIS A: GENERAL, ELSEVIER SCIENCE, AMSTERDAM, NL, vol. 256, no. 1-2, 30 December 2003 (2003-12-30), pages 3-18, XP004475295 ISSN: 0926-860X
- I.V.KOZHEVNIKOV: "Efficient acylation of toluene and anisole with aliphatic carboxylic acids catalysed by heteropoly salt Cs2,5H0,5PW12O40" CHEM.COMM., 2002, pages 2508-2509, XP002326740 cited in the application

## Description

The present invention provides a novel process for the preparation of aromatic thioether ketones. A process for the production of α-aminoketone photoinitiator compounds, employing said aromatic thioether compounds is disclosed.

The Friedel-Crafts acylation of aromatic compounds is the most important route for the synthesis of aromatic ketones, familiar to the person skilled in the art and published in many general textbooks of Chemistry. The acylation of aromatic ether or thioether compounds is known and described using different acylating agents and different types of acidic catalysts like stoichiometric AlCl₃ or toxic SnCl₄ as described for example in Sulfur Letter, 1991, 12(3), 123; US 5883267 and GB 2330833. The use of rare earth metal trifluormethanesulfonates is published in Bulletin of the Chemical Society of Japan, 2000, 73(10), 2325 and Synlett, 2002, 10, 1653. Copper trifluormethanesulfonates as catalysts are known from Tetrahedron, 2001, 57, 241. The use of toxic uranyl salts is disclosed in Journal of Molecular Catalysis A: Chemical, 2000, 164(1-2), 195. The use of HF is described in Journal of Organic Chemistry, 1991, 56(20), 5955 and EP 361790, while in Journal of Organic Chemistry, 1996, 61(26), 9546 alumina/trifluoroacetic anhydride is employed under microwave conditions. ZnCl₂ as catalyst is known from Indian Journal of Heterocyclic Chemistry, 2002, 11, 229.
Zeolite catalysts in Friedel Crafts reactions are for example disclosed in US 6608232, US 6121496, US 6194616, US 5817878, US 6013840, EP 459495, EP 334096, J. Molecular Catalysis: Chemical 1998, 134, 121, Applied Catalysis A: General, 2000, 201, 159, while the use of clays or exchanged clays is known from US 4304941 and EP 352878.
The application of heteropoly acids or heteropoly acid-containing solid supports for the acylation of aromatic ethers or aromatic hydrocarbons is described in Journal of Molecular Catalysis A: Chemical 2004, 209(1-2), 189; Chemical Communications 2002, 21, 2508; Journal of Catalysis 2002, 208, 448; Bulletin of the Chemical Society of Japan 1980, 53, 3389 and WO 03/101925.

The acylation of aromatic substrates is of great industrial interest for making aromatic intermediates used for the production of pharmaceuticals, insecticides, plasticizers, dyes, perfumes, and other commercial products, like photoinitiators.

In technique, in particular the preparation of aromatic thioether ketones as educts for photoinitiator compounds, there still exists a need for environmental friendly and economic ways to conduct the Friedel-Crafts Acylation.

It was now surprisingly found that the acylation of aromatic thioether compounds to give aromatic thioether ketones can very efficiently be conducted in the presence of heteropoly acids or heteropoly acid-containing solid supports.

Subject of the invention therefore is a process for synthesizing aromatic thioether ketones which comprises reacting an aromatic thioether with an acylating agent, selected from the group consisting of carboxylic acids, halides or anhydrides, in the presence of heteropoly acids or heteropoly acid-containing solid supports or the salts of heteropoly acids.

The new process avoids the use of stoichiometric amounts of toxic and corrosive catalysts, results in high yields of the aromatic thioether ketone compound and allows economic average utilizations of the production plant, because of shorter production times and shorter cycle times. Even solvent-less conditions can be chosen.

Suitable aromatic thioether compounds to be reacted in the process according to the present invention are, for example, compounds of the following formula (I') 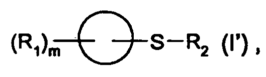 wherein
the circle represents an aromatic or heteroaromatic ring;
m is defined by the number of the C-atoms of the aromatic or heteraromatic ring minus 2;
R₁ represents one or more substituents, which may be identical or different;
R₂ represents a hydrocarbon radical containing 1 to 24 carbon atoms, which is a linear or branched saturated or unsaturated acyclic aliphatic radical, a saturated or unsaturated, monocyclic or polycyclic cycloaliphatic radical, a saturated or unsaturated, linear or branched aliphatic radical carrying cyclic substituents, an aromatic monocyclic or polycyclic radical; or R₁ and R₂ together form a cycle which optionally besides the sulfur atom comprises a further heteroatom.

The aromatic ring, represented by the circle in the above formula is for example phenyl, naphthyl, anthryl, phenanthryl etc.. The heteroaromatic ring may contain one or more heteroatoms, preferably one heteroatom, which atoms are for example O, S, P, N. Examples for heteroaromatic rings are furyl, thienyl, pyrrolyl, pyrridyl etc.. Further, the aromatic heterocycle may contain condensed rings, as for example benzofuryl, quinolyl, isoquinolyl etc..

The aromatic thioether to be reacted in the process according to the invention for example is of formula (I) 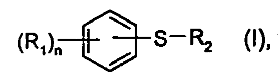 wherein
n is a number from 0 to 4;
R₁ represents one or more substituents, which are identical or different;
R₂ represents a hydrocarbon radical containing 1 to 24 carbon atoms, which is a linear or branched, saturated or unsaturated unsubstituted or substituted acyclic aliphatic radical; a saturated or unsaturated, monocyclic or polycyclic, unsubstituted or substituted cycloaliphatic radical; a saturated or unsaturated, linear or branched, unsubstituted or substituted aliphatic radical carrying cyclic substituents; or an unsubstituted or substituted aromatic monocyclic or polycyclic radical; or
R₁ and R₂ together form an unsubstituted or substituted cycle which optionally besides the sulfur atom comprises a further heteroatom.

R₁ is for example C₁-C₂₄alkyl, which optionally is substituted by halogen, CN, and/or OR₆; or is C₂-C₁₂alkenyl, C₃-C₈cycloalkyl; or is phenyl, optionally substituted by C₁-C₂₄alkyl, halogen, CN, and/or OR₆; or is naphthyl, halogen, OR₆, -CO-R₆, SR₆, NR₆R₇, or B(OR₆)(OR₇);
R₆ is hydrogen, C₁-C₂₄alkyl, C₁-C₂₄haloalkyl, and
R₇ has the same meanings as given for R₆.

Examples for linear or branched saturated or unsaturated acyclic aliphatic hydrocarbon radicals containing 1 to 24 carbon atoms are linear or branched C₁-C₂₄alkyl, linear or branched C₂-C₂₄alkenyl and linear or branched C₂-C₂₄alkynyl. E.g. C₁-C₁₈-, C₁-C₁₄-, C₁-C₁₂-, C₁-C₈-, C₁-C₆- or C₁-C₄alkyl. Examples are methyl, ethyl, propyl, isopropyl, n-butyl, sec-butyl, isobutyl, tert-butyl, pentyl, hexyl, heptyl, 2,4,4-trimethylpentyl, 2-ethylhexyl, octyl, nonyl, decyl, dodecyl, tetradecyl, pentadecyl, hexadecyl, octadecyl or icosyl.
C₁-C₁₈alkyl, C₁-C₁₄alkyl, C₁-C₁₂alkyl, C₁-C₈alkyl, C₁-C₆alkyl and C₁-C₄alkyl have the same meanings as given above for C₁-C₂₀alkyl up to the corresponding number of C-atoms.

The linear or branched C₂-C₂₄alkenyl radicals may be mono or polyunsaturated and are for example C₂-C₂₀-, C₂-C₁₈-, C₂-C₁₂-, C₂-C₈-, C₂-C₆- or C₂-C₄alkenyl. Examples are allyl, methallyl, vinyl, 1,1-dimethylallyl, 1-butenyl, 3-butenyl, 2-butenyl, 1,3-pentadienyl, 5-hexenyl or 7-octenyl, especially allyl or vinyl.
The linear or branched C₂-C₂₄alkynyl radicals may be mono or polyunsaturated and are for example C₂-C₂₀-, C₂-C₁₈-, C₂-C₁₂-, C₂-C₈-, C₂-C₆- or C₂-C₄alkynyl, e.g. propargyl, 1-propynyl, 2-propynyl.
Said linear or branched saturated or unsaturated acyclic aliphatic hydrocarbon radicals containing 1 to 24 carbon atoms are unsubstituted or substituted.
Examples for substituents are phenyl, phenyl substituted by halogen, NR₉R₁₀, alkyl and/or COOR₈; halogen, phenylthio, OR₈, N₃, CN, NR₉R₁₀, (CO)OR₈, (CO)R₁₂, Si(R₁₁)₃, or (PO)(OR₁₁)₂, wherein R₈ is hydrogen or C₁-C₁₂alkyl; R₉ and R₁₀ independently of one another are hydrogen, C₁-C₁₂alkyl or R₉ and R₁₀ together with the nitrogen atom to which they are attached form a ring, e.g. a piperidinyl ring, R₁₁ designates C₁-C₁₂alkyl and R₁₂ is phenyl, C₁-C₁₂alkyl or NR₉R₁₀.

Accordingly, the meaning linear or branched saturated or unsaturated acyclic aliphatic hydrocarbon radicals containing 1 to 24 carbon atoms, in the context of the present invention also corresponds to linear or branched C₁-C₂₄haloalkyl and linear or branched C₂-C₂₄haloalkenyl.
C₁-C₂₄Haloalkyl is for example C₁-C₁₂-, C₁-C₁₀-, C₁-C₈- or C₁-C₄-alkyl mono- or polysubstituted by halogen, the alkyl part of the molecule being, for example, as defined above. There are, for example, from one to three or one or two halogen substituents at the alkyl radical. Examples are chloromethyl, dichloromethyl, trichloromethyl, trifluoromethyl or 2-bromopropyl, especially trifluoromethyl, dichloromethyl or chloromethyl.
The definitions of C₂-C₂₄haloalkenyl correspond to the definitions given for alkenyl, wherein the alkenyl is substituted by one or more halogen as described for the alkyl groups.

Examples for saturated or unsaturated, monocyclic or polycyclic cycloaliphatic hydrocarbon radicals are C₃-C₂₄cycloalkyl radicals.
C₃-C₂₄Cycloalkyl is for example C₃-C₈Cycloalkyl, C₃-C₆- C₅- or C₆-Cycloalkyl, e.g. cyclopropyl, cyclopentyl, cyclohexyl, cyclooctyl, cyclododecyl, especially cyclopentyl and cyclohexyl, preferably cyclohexyl. Further encompassed by this definition are hydrocarbon rings bearing alkyl substituents as, for example methyl-cyclopentyl, methyl- or dimethylcyclohexyl, etc.. Said Cycloalkyl may consist of one ring only, but may also be a sytem of more than one rings, e.g. adamantyl.
Said saturated or unsaturated, monocyclic or polycyclic cycloaliphatic hydrocarbon radicals, i.e. the C₃-C₂₄cycloalkyl radicals are unsubstituted or substituted. Suitable substituents correspond to the ones as given above for the linear or branched saturated or unsaturated acyclic aliphatic hydrocarbon radicals containing 1 to 24 carbon atoms.

Examples for aromatic, monocyclic or polycyclic radicals are C₆-C₂₄aryl, e.g. C₆-C₁₀-aryl, as phenyl, 1-naphthyl, 2-naphthyl, anthryl, phenanthryl, biphenylyl, in particular phenyl.
Said aromatic, monocyclic or polycyclic radicals, i.e C₆-C₂₄aryl, are unsubstituted or substituted. Suitable substituents correspond to the ones as given above for the linear or branched saturated or unsaturated acyclic aliphatic hydrocarbon radicals containing 1 to 24 carbon atoms.

Examples for saturated or unsaturated, linear or branched aliphatic hydrocarbon radicals containing 1 to 24 carbon atoms, carrying cyclic substituents are C₁-C₂₄alkyl as defined above substituted by cycloalkyl or aryl, as defined above, up to the corresponding number of C-atoms; or are C₂-C₂₄alkenyl as defined above substituted by cycloalkyl or aryl, as defined above, up to the corresponding number of C-atoms. Examples are phenyl-C₁-C₁₈alkyl, like for example benzyl, phenylethyl, α-methylbenzyl, phenylpentyl, phenylhexyl or α,α-dimethylbenzyl; or naphthyl-C₁-C₁₄alkyl, like naphthylmethyl, naphthylethyl, naphthylpropyl or naphthyl-1-methylethyl
Said saturated or unsaturated, linear or branched aliphatic hydrocarbon radicals containing 1 to 24 carbon atoms, carrying cyclic substituents as defined above are unsubstituted or substituted. Suitable substituents correspond to the ones as given above for the linear or branched saturated or unsaturated acyclic aliphatic hydrocarbon radicals containing 1 to 24 carbon atoms.

If R₁ and R₂ together form a cycle which optionally besides the sulfur atom comprises a further heteroatom, e.g. S, O, NH or NR₂, in other words R₁ and R₂ together form an alkylene, which optionally is interrupted by a heteroatom. Said alkylene is linear or branched and suitably has about 2 to 10 atoms, for example 2 to 8 or 2 to 4 atoms, optionally interrupted by a heteroatom. For example the following structures are encompassed 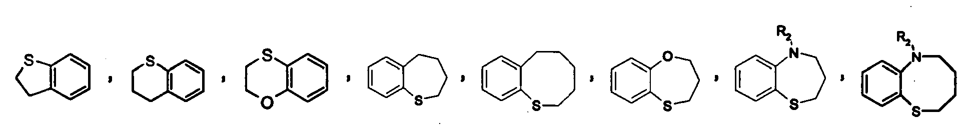 etc..
Said cycle which optionally besides the sulfur atom comprises a further heteroatom, is unsubstituted or substituted. Suitable substituents correspond to the ones as given above for the linear or branched saturated or unsaturated acyclic aliphatic hydrocarbon radicals containing 1 to 24 carbon atoms.

R₂ for example is C₁-C₂₄alkyl, C₁-C₂₄alkenyl, C₁-C₂₄alkynyl, C₃-C₂₄cycloalkyl, C₆-C₂₄aryl, phenyl-C₁-C₁₈alkyl or naphthyl-C₁-C₂₄alkyl, all which are unsubstituted or are substituted by phenyl, or substituted by phenyl substituted by halogen, NR₉R₁₀, alkyl and/or COOR₈; or are substituted by halogen, phenylthio, OR₈, N₃, CN, NR₉R₁₀, (CO)OR₈, (CO)R₁₂, Si(R₁₁)₃, or (PO)(OR₁₁)₂, wherein
R₈ is hydrogen or C₁-C₁₂alkyl;
R₉ and Rio independently of one another are hydrogen, C₁-C₁₂alkyl or R₉ and R₁₀ together with the nitrogen atom to which they are attached form a ring, e.g. a piperidinyl ring;
R₁₁ designates C₁-C₁₂alkyl and
R₁₂ is phenyl, C₁-C₁₂alkyl or NR₉R₁₀.
The meanings C₁-C₂₄alkyl, C₁-C₂₄alkenyl, C₁-C₂₄alkynyl, C₃-C₂₄cycloalkyl, C₆-C₂₄aryl, phenyl-C₁-C₁₈alkyl or naphthyl-C₁-C₂₄alkyl being defined as given above.

Interesting thioethers of formula I are such, wherein
R₁ is C₁-C₂₄alkyl, which optionally is substituted by halogen, CN, and/or OR₆;
or is C₃-C₈-cycloalkyl; or is phenyl, optionally substituted by C₁-C₂₄alkyl, halogen, CN, and/or OR₆; or is halogen, OR₆, -CO-R₆, SR₆, or NR₆R₇;
R₂ is linear or branched C₁-C₂₄alkyl, linear or branched C₂-C₂₄alkenyl;
C₃-C₂₄cycloalkyl, C₆-C₂₄aryl; or C₁-C₂₄alkyl substituted by C₃-C₈cycloalkyl or by C₆-C₁₀aryl; or
R₁ and R₂ together form C₁-C₂₄alkylene which optionally is interrupted by a heteroatom, or form C₂-C₂₄alkenylene, which optionally is interrupted by a heteroatom.
R₆ is hydrogen, C₁-C₂₄alkyl, C₁-C₂₄haloalkyl, and
R₇ has the same meanings as given for R₆.

In preferred compounds of formula I n is 0. In preferred compounds of formula I' m is 0.

R₁ in particular is C₁-C₂₄alkyl, e.g. C₁-C₄alkyl, halogen, OR₆, SR₆, or NR₆R₇.
R₂ is for example C₁-C₁₂- or C₁-C₈alkyl.

In particular suitable thioethers are thioanisole, ethyl phenyl sulfide, diphenyl sulfide, phenyl n-propyl sulfide, benzyl phenyl sulfide, phenyl β-phenylethyl sulfide, phenyl 3-phenylpropyl sulfide, allyl phenyl sulfide, phenyl propargyl sulfide, methallylphenyl sulfide, bis(phenylthio)methane, 1,3-bis(phenylthio)propane, 1,2-bis(phenylthio)ethane, 1,1,3-tris(phenylthio)-1-propene, chloromethyl phenyl sulfide, 2-chloroethyl phenyl sulfide, 3-chloropropyl phenyl sulfide, 2-bromoethyl phenyl sulfide, bromomethyl, phenyl sulfide, 4-fluorobenzyl phenyl, sulfide, 1-chloro-4-phenylsulfanyl benzene, 2-(phenylthio)ethanol, methoxymethyl phenyl sulfide, 2-methoxyethyl phenyl sulfide, azidomethyl phenyl sulfide, phenylthioacetonitrile, 1-[(phenylthio)methyl]piperidine, 4-[(phenylsulfanyl)methyl]aniline, (phenylthio)acetic acid, 3-(phenylsulfanyl)propanoic acid, 4-(phenylsulfanyl)butanoic acid, 2-phenylmercaptomethylbenzoic acid, methyl (phenylthio)acetate, ethyl (phenylthio)acetate, (phenylthiomethyl)trimethylsilane, 2-[(phenylthio)methyl]-2-cyclopenten-1-one, (phenylthio)-propanone, phenyl phenacyl sulfide, diethyl phenylthiomethylphosphonate, phenylthiomethyl, α-(phenylthio)acetamide, 3-(phenylthio)propionamide, 2-(phenylthio)ethanethioamide. In a preferred process, the thioether is thioanisole.

The person skilled in the art is familiar with the preparation of thioethers according to the formulae I' and I. Suitable preparation methods are given in general textbooks of chemistry, for example in "Advanced Organic Chemistry; Reactions, Mechanisms, and Strcutures; Fourth Edition, Jerry March; p.407-409."
A host of thioethers is also commercially available.

As acylating agent all agents usual in technique are suitable. Persons skilled in the art are familiar with such agents, many of which are commercially available. Usually said agents are selected from the group consisiting of acylhalides, carboxylic acids, carboxylic acid anhydrides.

The acylating agent is, for example, of formula (II) 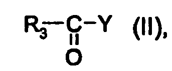 wherein
R₃ represents a linear or branched, saturated or unsaturated unsubstituted or substituted aliphatic radical containing 1 to 24 carbon atoms; a monocyclic or polycyclic saturated or unsaturated, unsubstituted or substituted cycloaliphatic radical containing 3 to 8 carbon atoms; or a linear or branched, saturated or unsaturated aliphatic radical carrying a cyclic substituent; or an aromatic monocyclic or polycyclic, unsubstituted or substituted radical containing 3 to 10 carbon atoms;
Y represents a halogen atom, a hydroxyl group, a group -O-COR₄, or -O-SO₂-R₄;
R₄ has one of the meanings given for R₃, or is C₁-C₂₄alkyl which is interrupted by one or more O and which optionally is substituted by halogen; or
R₃ and R₄ together form a divalent linear or branched, saturated or unsaturated aliphatic radical containing at least 2 carbon atoms.

R₃ and R₄ as a linear or branched, saturated or unsaturated unsubstituted or substituted aliphatic radical containing 1 to 24 carbon atoms; a monocyclic or polycyclic saturated or unsaturated, unsubstituted or substituted cycloaliphatic radical containing 3 to 8 carbon atoms; or a linear or branched, saturated or unsaturated aliphatic radical carrying a cyclic substituent; or an aromatic monocyclic or polycyclic, unsubstituted or substituted radical containing 3 to 10 carbon atoms;

Halogen corresponds to fluorine, chlorine, bromine and iodine, especially chlorine and bromine, preferably chlorine.

R₃ and R₄ for example are C₁-C₂₄alkyl substituted by one or more halogen. C₁-C₂₄alkyl substituted by one or more halogen, means C₁-C₂₀-, C₁-C₁₈-, C₁-C₁₂-, C₁-C₁₀-, C₁-C₈-, C₁-C₆- or C₁-C₄alkyl, which is linear or branched and defined as above and is substituted by one or more halogen, wherein the halogen atoms are either positioned at different C-atoms of the alkyl group or also at one C-atom. Examples are brommethyl, fluoromethyl, trifluoromethyl, fluoroethyl, chloromethyl, chloroethyl, pentafluoroethyl etc..The term C₁-C₂₄alkyl substituted by one or more halogen also encompasses the corresponding perhalogenated, in particular the perfluorinated alkyl.
C₁-C₂₄alkyl substituted by one or more halogen is for example Cₙ[HₓHal_{y}]₂ₙ₊₁, wherein the sum of x+y = 2n+1 and Hal, is halogen, preferably F. Examples are CF₃, C₂F₅, CH₂F, CHF₂, C₈F₁₇, C₄F₉, etc..
R₄ as C₁-C₂₄alkyl which is interrupted by one or more O and which optionally is substituted by halogen, is interrupted, for example, from one to five times, for example from one to three times or once or twice, by non-successive O. Accordingly, resulting structural units are for example: -OC₁-C₂₄alkyl, -O(CH₂)₂OH, -O(CH₂)₂OCH₃, -O(CH₂CH₂O)₂CH₂CH₃, -CH₂-O-CH₃, -CH₂CH₂-O-CH₂CH₃, -O[CH₂CH₂O]_{y}CH₃, -O[CH₂CH₂O]_{y}CH₂CH₃, -[CH₂CH₂O]_{y}-CH₃, -[CH₂CH₂O]_{y}-C₂H₅, wherein y = 1-5, -(CH₂CH₂O)₅CH₂CH₃, -CH₂-CH(CH₃)-O-CH₂-CH₂CH₃ or -CH₂-CH(CH₃)-O-CH₂-CH₃. If said alkyl interrupted by O is additionally substituted by one or more halogen, said halogen can replace one or more or all of the hydrogen atoms of the alkyl groups. Examples are -O[CF₂CF₂O]_{y}CF₃ , -O[CF₂CF₂O]_{y}CF₂CF₃, -[CF₂CF₂O]_{y}-CF₃, -[CF₂CF₂O]_{y}-C₂F₅, wherein y = 1-5, e.g. -CF₂CF₂-O-CF₂CF₃, -O-CF₂CF₂-O-CF₂CF₃.

R₃ and R₄ together as divalent linear or branched, saturated or unsaturated aliphatic radical containing at least 2 carbon atoms is for example alkylene or alkenylene. Said alkylene or alkenylene is linear or branched and suitably has about 2 to 10 atoms. For example C₂-C₁₀-, C₂-C₈-, C₂-C₄-, C₄-C₈-alkylene, C₂-C₁₀-, C₂-C₈-, C₂-C₄-, C₄-C₈-alkenylene. The specific meanings for said radicals correspond to the meanings given above for "alkyl" and "alkenyl" by adding the ending "ene" up to the corresponding number of C-atoms.

In interesting compounds of formula II Y is halogen atom, for example Cl, a hydroxyl group, or -O-COR₄.
R₃ is linear or branched C₁-C₂₄alkyl, linear or branched C₂-C₂₄alkenyl, linear or branched C₁-C₂₄haloalkyl, for example trifluormethyl, chloromethyl or dichloromethyl, linear or branched C₂-C₂₄haloalkenyl, C₃-C₈cycloalkyl, C₆-C₁₀aryl, i.e. phenyl or naphthyl; or are C₁-C₂₄alkyl substituted by C₃-C₈cycloalkyl or by C₆-C₁₀aryl.
R₄ has one of the meanings given for R₃, or is C₁-C₂₄alkyl substituted by one or more halogen or CN, or is C₁-C₂₄alkyl which is interrupted by one or more O and which optionally is substituted by halogen; or
R₃ and R₄ together form a divalent linear or branched, saturated or unsaturated aliphatic radical containing at least 2 carbon atoms.
Or R₃ and R₄ together form linear or branched C₂-C₁₀-alkylene, or linear or branched C₂-C₁₀alkenylene.

In particular compounds of formula II Y is -O-CO-R₄.
R₃ preferably is C₁-C₂₄alkyl, in particular C₁-C₁₂alkyl, for example methyl,ethyl, propyl or butyl; or is C₁-C₂₄haloalkyl, for example trifluoromethyl, monochlormethyl or dichloromethyl; or is phenyl.
Preferred are compounds wherein R₄ is identical to R₃.

A preferred acylating agent in the process according to the invention is selected from the group consisting of acetic anhydride, propanoic anhydride, butyric anhydride, *i*-butyric anhydride, trifluoroacetic anhydride, benzoic anhydride, monochloroacetic anhydride, dichloroacetic anhydride.
In a preferred process the acylating agent is *i*-butyric anhydride.

The person skilled in the art is familiar with the preparation of acylating agent according to the formula II. Suitable preparation methods are given in general textbooks of chemistry, for example in "Advanced Organic Chemistry; Reactions, Mechanisms, and Strcutures; Fourth Edition, Jerry March; p.401-402", in particular a description for the case, if Y= -OSO₂-R₄: "F. Effenberger et al., Angew. Chem. 1972, 84, 294."
A host of acylating agents is also commercially available.

Interesting is a process for synthesizing aromatic thioether ketones by reacting an aromatic thioether of the formula (I) according to claim 2 with an acylating agent of the formula (II) according to claim 4.

The terms "and/or" or "or/and" in the present context are meant to express that not only one of the defined alternatives (substituents) may be present, but also several of the defined alternatives (substituents) together, namely mixtures of different alternatives (substituents). The term "at least" is meant to define one or more than one, for example one or two or three, preferably one or two.
The term "optionally substituted" means, that the radical to which it refers is either unsubstituted or substituted.
Throughout this specification and the claims which follow, unless the context requires otherwise, the word "comprise", or variations such as "comprises" or "comprising", will be understood to imply the inclusion of a stated integer or step or group of integers or steps but not the exclusion of any other integer or step or group of integers or steps.

Heteropoly acids or heteropoly acid-containing solid supports or the corresponding salts of heteropoly acids are known in the literature, see for example Chem. Rev. 1998, 98,1, 171-198; Chem. Commun., 2001, 1141. Said acids are complex proton acids that incorporate polyoxometalate anions having metal-oxygen octahedral as the basic structural units, as for example described in Ref. 17-24 in Chem. Rev. 1998, 98,1, 171-198.
The Keggin type heteropolyanion typically represented by the formula XM₁₂O₄₀^{*x*-8} wherein X is the central atom (for example Si, P), x is the oxidation state, and M is the metal ion (for example Mo, W) is the best known. Other polyoxometalates are represented by the formulae X₂M₁₈O₆₂^{2*x*-16} (Dawson structure), XM₁₁O₃₉^{*x*-12}, X₂M₁₇O₆₁^{2*x*-20} (Keggin and Dawson lacunary anions). The salts of heteropoly acids are containing cations from the groups 1-13, cations from the lanthanides and are containing ammonium cations as well, see for example Chem. Rev. 1998, 98,1, 171-198.

In particular suitable in the process according to the invention are heteropolyacids with Keggin type anions.

Central atoms in the heteropolyacid, besides P and Si, as already named above, are for example B, Ge or As.
As coordinating atom the heteropolyacid comprises Mo or W or a mixture thereof.

Accordingly a preferred catalyst is a heteropoly acid or a heteropoly acid-containing solid support or a salt of the heteropoly acid or a mixture thereof, wherein the heteropoly acid has a central atom comprising P, Si, B, Ge, As and a coordinating atom comprising Mo or W or a mixture thereof.

In the process according to the invention the heteropoly acid can be employed as such or on a suitable support. Examples for said support are silica, mesoporous molecular sieves, carbon, hydrotalcite-type anion clays or aluminum oxides (ALOX).
Interesting as support are silica, aluminum oxides, mesoporous molecular sieves and hydrotalcite type anion clays, especially silica, aluminum oxides and mesoporous molecular sieves. Preferred are silica and aluminum oxides (ALOX), in particular silica.

As already mentioned above, a wide variety of the cations can be selected for the heteropoly acid. In particular suitable examples are Li, Na, K, Rb, Cs, Mg, Ca, Fe, La, Ce, Cu, Zn, Al, ammonium, or mixtures thereof.

Examples of suitable heteropoly acid catalysts are published in Chem. Rev. 1995, 559-614, p.591, p.595, p.596, p.597; Chem. Rev. 1998, 359-387, p.362, p.373; Y. Izumi, K. Urabe, M. Onaka in Zeolite, Clay, and Heteropoly Acid; VCH Publishers Inc., New York, 1992; Chap. 3, p.111, p.141 (supported systems), p.154; Applied Catalysis A: General 132, 1995, 127-140, p.139; The Chemical Engineering Journal, 1996, 64, 247-254; p. 252; Chem. Commun., 2001, 1141-152, p.1144, p.1150; J. Molecular Catalysis A; Chemical 219, 2004, 327-341, p.331, p.332, p.334, p.336; Chem. Rev. 1998, 98, 307-325; J. Molecular Catalysis A: Chemical 219 (2004), 327-342; Applied Catalysis A: General 257 (2004), 19-23; J. Catalysis 208 (2002), 448-455; Microporous and Mesoporous Materials 21 (1998), 227-233.

Suitable are catalysts in the anhydrous form as well as the corresponding hydrated forms or the corresponding salts. Further, as already mentioned, the catalyst can be fixed on a support, in different loadings.
Specific examples of suitable catalysts are
K_{5.5}H_{1.5}[SbW₆O₂₄] ·6H₂O; Na₅[IMo₆O₂₄]·3H₂O; K₆[Mo₇O₂₄]·4H₂O; Cs₄[W₁₀O₃₂]·4H₂O; Cs₄[W₁₀O₃₂]·5H₂O; K₅[BW₁₂O₄₀]·15H₂O; K₃[PMo₁₂O₄₀]·4H₂O; K₃[PMo₁₂O₄₀]·5H₂O; [N(C₄H₉)₄]₂[W₆O₁₉]; [N(C₄H₉)₄]₂[Mo₆O₁₉]; K₉H₅[(GeTi₃W₉O₃₇)₂O₃] ·16H₂O; K_{2.5}H_{0.5}PW₁₂O₄₀; Cs_{2.5}H_{0.5}PW₁₂O₄₀; Rb_{2.5}H_{0.5}PW₁₂O₄₀; CS₃HiW₁₂O₄₀; K₂H₂SiW₁₂O₄₀; Mg₃[PMo₁₂O₄₀]₂; Ca₃[PMo₁₂O₄₀]₂; Sr₃[PMo₁₂O₄₀]₂; Ba₃[PMo₁₂O₄₀]₂; Li₃[PMo₁₂O₄₀]; Na₃[PMo₁₂O₄₀]; Al[pMo₁₂O₄₀]; H₃[PMo₁₂O₄₀]; H₃[PMo₁₂O₄₀]·28H₂O; Li₃[PMo₁₂O₄₀]; Li₃[PMo₁₂O₄₀]·29H₂O; Na₃[PMo₁₂O₄₀]; Na₃[PMo₁₂O₄₀]·29H₂O; Mg₃[PMo₁₂O₄₀]; Mg₃[PMo₁₂O₄₀]·58H₂O; Ca₃[PMo₁₂O₄₀]; Ca₃[PMo₁₂O₄₀]·58H₂O; Sr₃[PMo₁₂O₄₀]; Sr₃[PMo₁₂O₄₀]·58H₂O; Ba₃[PMo₁₂O₄₀]; Ba₃[PMo₁₂O₄₀]·58H₂O; Al₃[PMo₁₂O₄₀]; Al[PMo₁₂O₄₀]·29H₂O; H_{0.5}K_{2.5}PW₁₂O₄₀; H_{0.5}Na_{2.5}PW₁₂O₄₀; H_{0.5}Mg_{1.25}PW₁₂O₄₀; H₄GeMo₁₂O₄₀; (NH₄)₆P₂Mo₁₈O₆₂; [C₁₆H₃₃N(CH₃)₃]ₓH₄₋ₓSiMo₁₂O₄₀; [C₁₆H₃₃N(CH₃)₃]ₓH₄₋ₓSiW₁₂O₄₀; (NH₄)₁₀H₂W₁₂O₄₂; (NH₄)₆P₂W₁₂O₆₂; [C₁₆H₃₃N(CH₃)₃]₆Mo₇O₂₄; [(C₄H₉)₄N]₃PW₁₂O₄₀; [C₁₈H₃₇N(CH₃)₂C₂H₅]₁₀[H₂W₁₂O₄₀]; H₆P₂W₁₈O₆₂; H₃PW₁₂O₄₀.nH₂O; H₃PMo₆W₆O₄₀; (NH₃)₆[H₂W₁₂O₄₀]; H₃PW₁₂O₄₀; H₃PMo₁₂O₄₀·30H₂O; H₃PW₁₂O₄₀·30H₂O; (NH₄)₃PMo₁₂O₄₀·3H₂O; K₃PW₁₂O₄₀·6H₂O; Na₃PMo₁₂O₄₀·21 H₂O; (NH₄)₃PW₁₂O₄₀·3H₂O; K₃PMo₁₂O₄₀·6H₂O; Na₃PW₁₂O₄₀·21 H₂O; H₄SiMo₁₂O₄₀·26H₂O; Na₄SiMo₁₂O₄₀·22H₂O; H₄SiW₁₂O₄₀·30H₂O; Na₄SiW₁₂O₄₀·22H₂O; H₅BW₁₂O₄₀·30H₂O; K₆P₂W₁₈O₆₂·14H₂O;K₁₀P₂W₁₇O₆₁·22H₂O; (NH₄)₆TeMo₆O₂₄·7H₂O; (NH₄)₈CeMo₁₂O₄₂·8H₂O; (NH₄)₆MnMo₉O₃₂·8H₂O; Na₅W₆O₂₄·8H₂O; (CN₃H₆)₆TeW₆O₂₄·7H₂O; (NH₄)₃H₄AsW₁₂O₄₂·4H₂O; H₄GeW₁₂O₄₀·30H₂O; K₂SeMo₆O₂₁·6H₂O; Na₃AlMo₆O₂₁·11H₂O; K₄ZrMo₁₂O₄₀·18H₂O; La₃[PMO₁₂O₄₀]·10H₂O; Co₃[PMo₁₂O₄₀]₂· 34H₂O; Ni₃[PMo₁₂O₄₀]₂·34H₂O; Mn₃[PMo₁₂O₄₀]₂·41H₂O; Co₂[PMo₁₂O₄₀]·24H₂O; Ni₂[SiMo₁₂O₄₀]·21H₂O; Mn₂[SiMo₁₂O₄₀]·22H₂O; Cu₂[SiMo₁₂O₄₀]·20H₂O; La₄[SiMo₁₂O₄₀]·71H₂O; H₃PW₁₂O₄₀; H₄SiW₁₂O₄₀; H₄GeW₁₂O₄₀;H₅BW₁₂O₄₀; H₆CoW₁₂O₄₀; H₆P₂W₁₈O₆₂; H₄SiMo₁₂O₄₀; H₃PMo₁₂O₄₀; K_{2.6}H_{0.4}PW₁₂O₄₀; Cs₂HPW₁₂O₄₀; (NH₄,Cs,H)₃PW₁₂O₄₀; and some examples supported by molecular sieves are H₄SiW₁₂O₄₀/MCM-41; H₃PW₁₂O₄₀/MCM-41; or supported by silicates Cs_{2.5}H_{0.5}PW₁₂O₄/SiO₂ powder; Cs_{2.5}H_{0.5}PW₁₂O₄/SiO₂ extrudate; 50% H₃PW₁₂O₄₀/SiO₂; 40% H₃PW₁₂O₄₀/SiO₂; 30% H₃PW₁₂O₄₀/SiO₂; 20% H₃PW₁₂O₄₀/SiO₂; 10% H₃PW₁₂O₄₀/SiO₂; 40% H₄SiW₁₂O₄₀/SiO₂; 40% H₄SiW₁₂O₄₀/MCM-41; 40% H₃PMo₁₂O₄/SiO₂; 40% H₃PMo₁₂O₄/MCM-41;

Preferably the process is carried out using H₄Si(W₃O₁₀)₄, or H₃P(W₃O₁₀)₄ as the heteropoly acids catalyst.

The person skilled in the art is familiar with the preparation of such heteropoly acids. Suitable preparation methods are given in general textbooks of chemistry or for example in the literature named above, e.g. in "Y. lzumi, K. Urabe, M. Onaka in Zeolite, Clay, and Heteropoly Acid; VCH Publishers Inc., New York, 1992." Heteropoly acids are also commercially available in a wide variety.

The process according to the present invention can for example be described by the following general reactions: 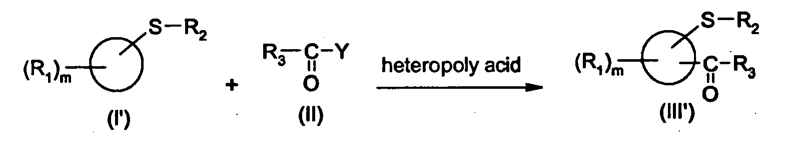 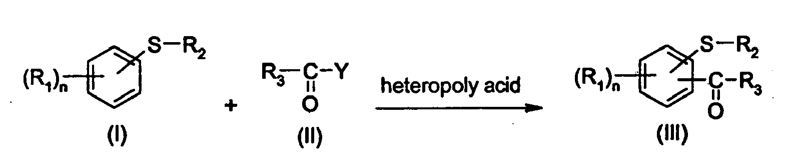 wherein R₁, R₂, R₃, Y n and m are as defined above.

The process is generally carried out by mixing the thioether educt with the acylating agent and reacting said educts in a suitable vessel, which is provided with a heating means. The reaction conveniently is carried out under inert conditions, i.e. the vessel should be equipped with appropriate means to create said atmosphere by for example working in an atmosphere of nitrogen. Other inert gases, as for example He, could also be employed. The person skilled in the art is familiar with these facts.

The reaction of the thioether of formula I or I' with the acylating agent of formula II can be carried out in different manner. Representative, but not exclusive examples are given below.
a) the thioether is placed, together with the catalyst and the acylating agent, in the reaction vessel and is immediately heated to the final reaction temperature, or
b) the thioether, together with the catalyst and the acylating agent, is placed in the reaction vessel and heated slowly during the reaction to the final temperature, or
c) the acylating agent is added during the reaction, to the thioether and the catalyst which have been previously heated to the reaction temperature,
d) the catalyst is suspended in a minimum amount of either one or both of the starting materials and then the reactants are added subsequently in any order or are added together.
The reaction vessel also may for example consist of a column that is filled with the catalyst and the acylating agent and the thioether are pumped (e.g. continuouesly) over the catalyst through the column.
A further possibility is to bring the reactants together via a reactive distillation, which is a process in which a catalytic chemical reaction and distillation occur simultaneously in a single apparatus.

Further solvents are, in principle, not required for carrying out the reaction, but may additionally be used. It is evident that the solvent must be inert under the respective reaction conditions. The person skilled in the art is familiar with suitable solvents for such reactions. Convenient solvents have been found to be for example aliphatic hydrocarbons (petrolethers), ethers, such as dioxane, tetrahydrofurane etc..

The molar ratios of the educts in the process according to the invention are not critical. Either an excess of the aromatic thioether or an excess of the acylating agent may be used. In the process according to the invention the mol ratio of the aromatic thioether to the acylating agent is for example from 1:10, 1:5, 1:2, or 1:1 or vice versa the mol ratio of the acylating agent to the aromatic thioether compound is from 1:10, 1:5, 1:2, or 1:1.

Interesting therefore is a process, wherein a molar excess of the aromatic thioether is employed; and a process, wherein a molar excess of the acylating agent is employed.

Subject of the invention also is a process, wherein the mol ratio of the aromatic thioether to the acylating agent is from 1 to 10; as well as a process, wherein the mol ratio of the acylating agent to the aromatic thioether is from 1 to 10.

The amount of the heteropolyacid catalyst can vary broadly. The catalyst is advantageously applied in an amount between 50 wt% to 0.01 wt%, for example between 25 wt% to 0.05 wt%, in particular 10 wt% to 0.1wt%.

The reaction temperatures in principle depent on the boiliong point of the educts and solvents that are employed in the reaction. Said temperature is conveniently in the range from room temperature to about 140°C, for example from 25°C to 140°C or from 25°C to 100°C, in particular from 40°C to 100°C, preferably from 40°C to 80°C.

The processing and purification of the novel phenyl alkyl ketones of formula III or III', which are substituted by a cyclic amine, is carried out by known methods, typically by distillation, crystallisation and filtration.

In particular interesting is a process according to the invention for synthesizing 2-methyl-1-[4-(methylthio)phenyl]-1-propanone by reacting thioanisole with *i*-butyric anhydride in the presence of heteropoly acids or heteropoly acid-containing solid supports or the corresponding salts of heteropoly acids.

The aromatic thioether ketones prepared according to a process of the invention are for example used to synthezise α-amino ketone photoinitiator compounds of formula (IV) or (IV') by introducing a corresponding amine group to the aromatic thioether ketone. This is for example done by first halogenating the aromatic thioether ketone compound and subsequently reacting the compound with a primary or secondary amine in the presence of a Lewis acid, as is for example described in WO 02/42254 and in EP-A-0003002 (method H): 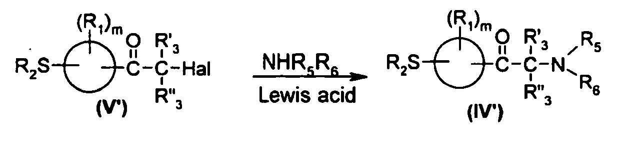 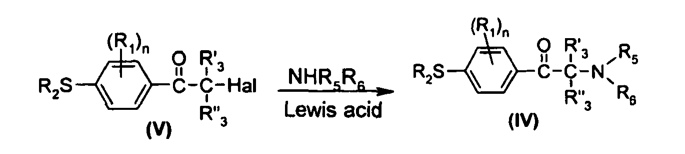 R'₃ and R"₃ are groups remaining from R₃, as defined above, after the halogenation, in particular methyl, i.e. R₃ is C₁-C₂₄haloalkyl.
R₁, n and R₂ have the same meaning as given above, in particular R₂ is methyl; R₅ and R₆ independently of one another are C₁-C₁₂alkyl, optionally substituted by hydroxy; phenyl, benzyl, or R₅ and R₆ together with the N-atom to which they are attached form an C₄-C₅-alkylene, which optionally is interrupted by a further N- or O-atom, e.g. a morpholino group.
In particular R₅ and R₆ together with the N-atom to which they are attached, form a morpholino group. Hal, is a halogen atom, in particular Cl or Br.

Another possibility is to react the halogenated aromatic thioether compound (V') or (V) with an alcoholate, such as for example sodium methoxylate, and subsequently with a primary or secondary amine to give the α-amino ketone compound (IV) or (IV'), as is disclosed for example in EP 3002: 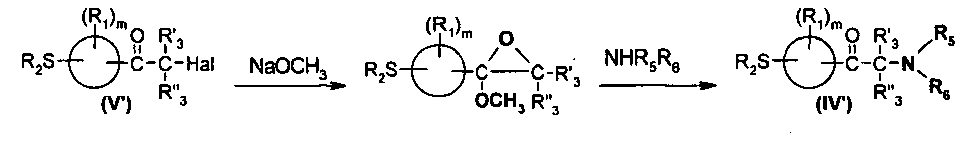 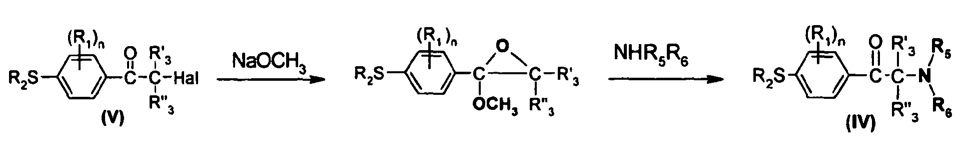 The meanings for Hal, R₁, n, R₂, R'₃, R"₃, R₅ and R₆ are as given above.

A process for synthesizing an α-amino ketone photoinitiator compound is dislosed, which comprises reacting an aromatic thioether ketone obtained by a process as described above with a halogene, in particular chlorine or bromine, reacting the obtained halogenated compound either
(a) with a primary or secondary amine in the presence of a Lewis acid;
   or
(b) with an alkali alcoholate, in particular sodium methoxylate, and subsequently with a primary or secondary amine,
to give the α-amino ketone photoinitiator compound.

Interesting therefore is a process for synthesizing an α-amino ketone photoinitiator compound of formula (IV) 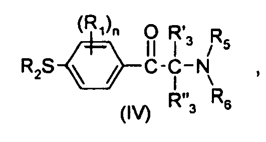 wherein R₁, R₂, n, R₃', R₃", R₅ and R₆ are as defined above;
which comprises reacting an aromatic thioether ketone of the formula (III) 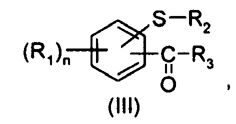 wherein R₁, R₂, n and R₃ are as defined above;
obtained by reacting a thioether of the formula (I) 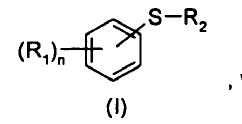 wherein R₁, R₂, and n are as defined above;
with an acylating agent of the formula (II) 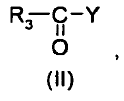 wherein R₃ is as defined above;
with a halogene, in particular chlorine or bromine, reacting the obtained halogenated compound either
(a) with a primary or secondary amine in the presence of a Lewis acid;
   or
(b) with an alkali alcoholate, in particular sodium methoxylate, and subsequently with a primary or secondary amine.

Further disclosed is the use of aromatic thioether compounds, obtained by a process as described above, for the preparation of α-amino ketone photoinitiator compounds.
A photopolymerizable composition is disclosed comprising
(A) at least one compound comprising an ethylenically unsaturated component, and
(B) a photoinitiator compound obtained by the process as described above,
where the composition, in addition to the component (B), can also comprise other photoinitiators (C) and/or other additives (D).

Suitable ethylenically unsaturated compounds for a radically photopolymerizable formulation are known to the person skilled in the art and published in a host of patents, e.g. WO 03/091287.
The unsaturated compounds can contain one or more olefinic double bonds. They can be of low molecular weight (monomeric) or relatively high molecular weight (oligomeric).
The photopolymerizable compounds may be used on their own or in any desired mixtures. Preference is given to using mixtures of polyol (meth)acrylates.
It is also possible to add binders to the compositions according to the invention; this is particularly advantageous if the photopolymerizable compounds are liquid or viscose substances.
Apart from the photoinitiator, the photopolymerizable mixtures can also contain various additives (D). Examples thereof are known to the person skilled in the art, e.g. optical brighteners, fillers, pigments, both white and coloured pigments, dyes, antistats, wetting agents or levelling auxiliaries, thermal inhibitors, additives to increase the storage stability in the dark, light protection agents which may be used are UV absorbers, for example those of the hydroxyphenylbenzotriazol, hydroxyphenylbenzophenone, oxalamide or hydroxyphenyl-s-triazine type. The compounds can be used individually or as mixtures, with or without the use of sterically hindered amines (HALS).
Further additives (D) are levelling auxiliaries, amines, chain transfer reagents, adhesion improvers, thiols, thioethers, disulfides and phosphines, photosensitizers, that shift and/or broaden the spectral sensitivity, e.g. benzophenone, thioxanthone, anthraquinone and 3-acylcoumarin derivatives, terphenyls, styryl ketones, and 3-(aroylmethylene)thiazolines, camphorquinone, but also eosin, rhodamine and erythrosine dyes.
The choice of additives depends on the field of application in question and the properties desired for this field. The above-described additives (D) are customary in the art and are accordingly used in amounts customary in the art.
The composition may also comprise further photointiators (C), known to the art-skilled person, e.g. benzophenone and derivatives thereof, acetophenone and derivatives thereof, 4-aroyl-1,3-dioxolanes, benzoin alkyl ethers and benzil ketals, for example benzil dimethyl ketal, phenyl glyoxalates and derivatives thereof, dimeric phenyl glyoxalates, peresters, e.g. benzophenonetetracarboxylic peresters, monoacylphosphine oxides, for example (2,4,6-trimethylbenzoyl)phenylphosphine oxide, bisacylphosphine oxides, for example bis(2,6-dimethoxybenzoyl)(2,4,4-trimethylpent-1-yl)phosphine oxide, bis(2,4,6-trimethylbenzoyl)-phenylphosphine oxide or bis(2,4,6-trimethylbenzoyl)(2,4-dipentoxyphenyl)phosphine oxide, trisacylphosphine oxides, halomethyltriazines, e.g. 2-[2-(4-methoxyphenyl)vinyl]-4,6-bistrichloromethyl-[1,3,5]triazine, 2-(4-methoxyphenyl)-4,6-bis-trichloromethyl-[1,3,5]triazine, 2-(3,4-dimethoxyphenyl)-4,6-bistrichloromethyl-[1,3,5]triazine, 2-methyl-4,6-bistrichloromethyl-[1,3,5]triazine, hexaarylbisimidazole/coinitiator systems, e.g. ortho-chlorohexaphenylbisimidazole in combination with 2-mercaptobenzothiazole; ferrocenium compounds or titanocenes, for example dicyclopentadienylbis(2,6-difluoro-3-pyrrolophenyl)titanium. Coinitiators which may also be used are borate compounds.

The photopolymerizable compositions advantageously comprise the photoinitiator in an amount of from 0.05 to 20% by weight, e.g. 0.05 to 15% by weight, preferably 0.1 to 5% by weight, based on the composition. The amount of photoinitiator stated is based on the total of all added photoinitiators if mixtures thereof are used, i.e. both on the photoinitiator (B) and on the photoinitiators (B) + (C).

The photopolymerizable compositions can be used for various purposes, for example as printing inks, such as screen printing inks, flexographic printing inks or offset printing inks, as clearcoats, as colour coats, as white coats, e.g. for wood or metal, as powder coatings, as paints, for photographic reproduction processes, for holographic recording materials, for image recording processes or for the production of printing plates, for the production of masks for screen printing, as dental filling materials, as adhesives, as pressure-sensitive adhesives, as laminating resins, as photoresists, e.g. galvanoresists, etch or permanent resists, both liquid and dry films, as photostructurable dielectrics, as solder stopping masks for electronic circuits, as resists for the preparation of colour filters for any type of screen or for producing structures in the production process of plasma displays and electroluminescence displays, for the production of optical switches, optical gratings (interference gratings), for the preparation of three-dimensional objects by mass curing (UV curing in transparent moulds) or by the stereolithography process, for the preparation of composite materials and other thick-layer materials, for the preparation of gel coats, for the coating or sealing of electronic components or as coatings for optical fibres. The compositions are also suitable for the preparation of optical lenses, e.g. contact lenses and Fresnel lenses, and for the preparation of medical instruments, auxiliaries or implants.
The person skilled in the art is well aware of said variuos uses for radically polymerizable compositions.

The following Examples further illustrate the invention but it is not intended to limit the invention to the Examples. Here, as in the remainder of the description and in the claims, parts and percentages relate to weight, unless otherwise indicated. If alkyl groups or alkoxy groups are named without specifying their isomeric forms, the n-isomer is meant.

The reactions are run in a Radley Carousel Reaction Station™ using 25 ml glass reaction tubes with gas-tight caps under inert atmosphere, by using nitrogen.

### Example 1:

Preparation of

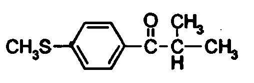

4.47 g (36 mmol) of thioanisole and 1.89 g (12 mmol) of *i*-butyric anhydride are placed in the glass reaction tube. Then 0.19 g of H₄Si(W₃O₁₀)₄ are added to the reaction mass and the glass reaction tube is fitted with the gas-tight cap. The glass reaction tube is heated to 80°C. Gas chromatographic analysis (GC) reveals 94% of product (based on *i*-butyric anhydride) formed after 3h.

### Example 2:

The procedure of example 1 is repeated but the H₄Si(W₃O₁₀)₄ is replaced by 0.19 g of H₃P(W₃O₁₀)₄. GC analysis reveals 93% of product (based on *i*-butyric anhydride) formed after 3h.

### Example 3:

1.49 g (12 mmol) of thioanisole and 5.69 g (36 mmol) of *i*-butyric anhydride are placed in the glass reaction tube. Then 0.57 g of H₄Si(W₃O₁₀)₄ are added to the reaction mass and the glass reaction tube is fitted with the gas-tight cap. The glass reaction tube is heated to 80°C. GC analysis reveals 68% of product (based on *i*-butyric anhydride) formed after 3h.

### Example 4:

The procedure of example 3 is repeated but the H₄Si(W₃O₁₀)₄ is replaced by 0.19 g of H₃P(W₃O₁₀)₄. GC analysis reveals 76% of product (based on *i*-butyric anhydride) formed after 3h.

### Example 5:

The procedure of example 1 is repeated, but the glass reaction tube is heated to 45°C. GC analysis reveals 80% of product (based on *i*-butyric anhydride) formed after 3h.

### Exampe 6:

The procedure of example 2 is repeated, but the glass reaction tube is heated to 45°C. GC analysis reveals 79% of product (based on *i*-butyric anhydride) formed after 3h.

### Example 7:

23.5 g (0.15 mol) of isobutyric anhydride and 56.0 g (0.45 mol) of thioanisole are placed in a 250 ml flask. 6 g of supported catalyst (= catalyst according to example 1 on SiO₂ support) are added portion wise to the reaction mixture. After complete addition the temperature is raised to 90°C (oil bath). After 23h at 90°C the GC analysis reveals 97.3% of product (based on *i*-butyric anhydride).

### Example 8:

### Synthesis of an α-aminoketone photo initiator, 2-methyl-1-[4-(methylthio)phenyl]-2-(4-morpholinyl)-1-propanone:

8.1: Chlorination 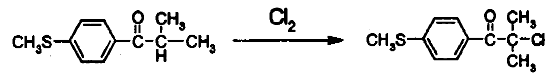 887 g of HCl (32%) are placed in a 2 L reactor. 500 g (2.55 mol) thioketone (99.0 %), prepared according to the method of example 1, are added drop wise using a dropping funnel. After complete addition the funnel is washed with 22 g of xylene. At room temperature 184.4 g (2.6 mol) of chlorine gas are dosed over a period of 2h. The reaction mixture is stirred over night. Afterwards 420 g of xylene are added, after stirring for 15 minutes the aqueous phase is separated. To the organic phase are added 150 g of water and 110 g of sodium carbonate solution in order to adjust the pH to 7-8. Finally, the aqueous phase is separated and the organic solvent evaporated yielding 578.1 g (94.4%) of the chloro thioketone as yellow oil.
8.2: Epoxidation 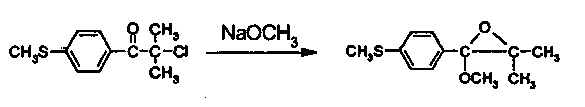 At room temperature 223 g of sodium methanolate solution are placed in a 1 L reactor and stirred. 250 g of chloro-thioketone, as prepared in 8.1, dissolved in 160 g of xylene are added drop wise within 3h using a dropping funnel. Afterwards the reaction mixture is headed to 50°C. 338 g of water are added. Finally, the aqueous phase is separated and the organic solvent evaporated yielding 241.8 g (96.2 %) of the epoxide as yellow oil.
8.3: Opening of the epoxy ring 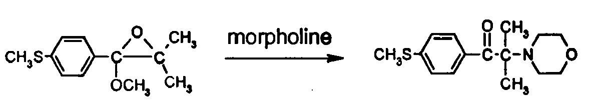 241.8 g of the epoxide, prepared in 8.2, are dissolved in 223 g of morpholine in a 1 L reactor at room temperature and mixed with 2.7 g of calcium hydroxide. The reaction mixture is heated to 125-138°C and the resulting methanol is continuously distilled off over a period of 20h. Afterwards the residual morpholine is evaporated yielding 294.4 g of the crude α-aminoketone photo initiator, which is crystallized from methanol giving 248.8 g (90.2 %) 2-methyl-1-[4-(methylthio)phenyl]-2-(4-morpholinyl)-1-propanone.

## Claims

1. A process for synthesizing aromatic thioether ketones which comprises reacting an aromatic thioether with an acylating agent, selected from the group consisting of carboxylic acids, halides or anhydrides, in the presence of heteropoly acids or heteropoly acid-containing solid supports or the salts of heteropoly acids.

2. A process according to claim 1, wherein the aromatic thioether is of the formula (I) 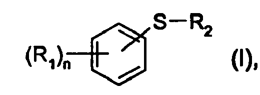 wherein
n is a number from 0 to 4;
R₁ represents one or more substituents, which are identical or different;
R₂ represents a hydrocarbon radical containing 1 to 24 carbon atoms, which is a linear or branched, saturated or unsaturated unsubstituted or substituted acyclic aliphatic radical; a saturated or unsaturated, monocyclic or polycyclic, unsubstituted or substituted cycloaliphatic radical; a saturated or unsaturated, linear or branched, unsubstituted or substituted aliphatic radical carrying cyclic substituents; or an unsubstituted or substituted aromatic monocyclic or polycyclic radical; or
R₁ and R₂ together form an unsubstituted or substituted cycle which optionally besides the sulfur atom comprises a further heteroatom.

3. A process according to claim 1, where the aromatic thioether compound is thioanisole, ethyl phenyl sulfide, diphenyl sulfide, phenyl n-propyl sulfide, benzyl phenyl sulfide, phenyl β-phenylethyl sulfide, phenyl 3-phenylpropyl sulfide, allyl phenyl sulfide, phenyl propargyl sulfide, methallylphenyl sulfide, bis(phenylthio)methane, 1,3-bis(phenylthio)propane, 1,2-bis(phenylthio)ethane, 1,1,3-tris(phenylthio)-1-propene, chloromethyl phenyl sulfide, 2-chloroethyl phenyl sulfide, 3-chloropropyl phenyl sulfide, 2-bromoethyl phenyl sulfide, bromomethyl phenyl sulfide, 4-fluorobenzyl phenyl sulfide, 1-chloro-4-phenylsulfanyl benzene, 2-(phenylthio)ethanol, methoxymethyl phenyl sulfide, 2-methoxyethyl phenyl sulfide, azidomethyl phenyl sulfide, phenylthioacetonitrile, 1-[(phenylthio)methyl]piperidine, 4-[(phenylsulfanyl)methyl]aniline, (phenylthio)acetic acid, 3-(phenylsulfanyl)propanoic acid, 4-(phenylsulfanyl)butanoic acid, 2-phenylmercaptomethylbenzoic acid, methyl (phenylthio)acetate, ethyl (phenylthio)acetate, (phenylthiomethyl)trimethylsilane, 2-[(phenylthio)methyl]-2-cydopenten-1-one, (phenylthio)propanone, phenyl phenacyl sulfide, diethyl phenylthiomethylphosphonate, phenylthiomethyl, α-(phenylthio)acetamide, 3-(phenylthio)propionamide, 2-(phenylthio)ethanethioamide.

4. A process according to claim 1, where the acylating agent is of the formula (II): 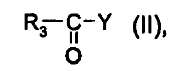 wherein
**R₃** represents a linear or branched, saturated or unsaturated unsubstituted or substituted aliphatic radical containing 1 to 24 carbon atoms; a monocyclic or polycyclic saturated or unsaturated, unsubstituted or substituted cycloaliphatic radical containing 3 to 8 carbon atoms; or a linear or branched, saturated or unsaturated aliphatic radical carrying a cyclic substituent; or an aromatic monocyclic or polycyclic, unsubstituted or substituted radical containing 3 to 10 carbon atoms;
**Y** represents a halogen atom, a hydroxyl group, a group -O-COR₄, or -O-SO₂-R₄;
**R₄** has one of the meanings given for R₃, or is C₁-C₂₄alkyl which is interrupted by one or more O and which optionally is substituted by halogen; or
**R₃** and **R₄** together form a divalent linear or branched, saturated or unsaturated aliphatic radical containing at least 2 carbon atoms.

5. A process according to claim 1, wherein the acylating agent is selected from the group consisting of acetic anhydride, propanoic anhydride, butyric anhydride, *i*-butyric anhydride, trifluoroacetic anhydride, benzoic anhydride, monochloroacetic anhydride, dichloroacetic anhydride.

6. A process according to claim 1, wherein the catalyst is a heteropoly acid or a heteropoly acid-containing solid support or a salt of the heteropoly acid or a mixture thereof, wherein the heteropoly acid has a central atom comprising P, Si, B, Ge, As and a coordinating atom comprising Mo or W or a mixture thereof.

7. A process according to claim 1, wherein the heteropoly acid is supported by silica, mesoporous molecular sieves, carbon, hydrotalcite-type anion clays or aluminum oxides.

8. A process according to claim 1, wherein the salt of the heteropoly acid comprises Li, Na, K, Rb, Cs, Mg, Ca, Fe, La, Ce, Cu, Zn, Al, ammonium or mixtures thereof.

9. A process according to claim 1, wherein the reaction temperature ranges from room temperature to 140°C, preferably from 40°C to 80°C.

10. A process according to claim 1, wherein the amount of heteropoly acid or heteropoly acid-containing solid support or salt of the heteropoly acid or mixture thereof is between 50wt% to 0.01wt%, preferably between 10wt% to 0.1wt%.

11. A process according to claim 1, wherein a molar excess of the aromatic thioether is employed.

12. A process according to claim 1, wherein a molar excess of the acylating agent is employed.

13. A process according to claim 1, wherein the mol ratio of the aromatic thioether to the acylating agent is from 1 to 10.

14. A process according to claim 1, wherein the mol ratio of the acylating agent to the aromatic thioether is from 1 to 10.

15. A process for synthesizing aromatic thioether ketones by reacting an aromatic thioether of the formula (I) according to claim 2 with an acylating agent of the formula (II) according to claim 4.

16. A process according to anyone of claims 1 to 15, wherein the aromatic thioether is thioanisole.

17. A process according to anyone of claims 1 to 16, wherein the acylating agent is *i*-butyric anhydride.

18. A process according to anyone of claims 1 to 17, where the heteropoly acids is H₄Si(W₃O₁₀)₄, or H₃P(W₃O₁₀)₄.

19. A process according to anyone of claims 1 to 18, for synthesizing 2-methyl-1-[4-(methylthio)phenyl]-1-propanone by reacting thioanisole with *i*-butyric anhydride in the presence of heteropoly acids or heteropoly acid-containing solid supports or the corresponding salts of heteropoly acids.

## Patentansprüche

1. Verfahren zum Synthetisieren aromatischer Thioetherketone, das Umsetzen eines aromatischen Thioethers mit einem Acylierungsmittel, ausgewählt aus der Gruppe, bestehend aus Carbonsäuren, -Halogeniden oder -Anhydriden, in Gegenwart von Heteropolysäuren oder Heteropolysäure enthaltenden festen Trägern oder den Salzen der Heteropolysäuren, umfasst.

2. Verfahren nach Anspruch 1, wobei der aromatische Thioether die Formel (I) 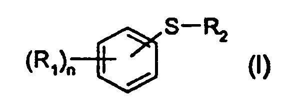 aufweist, worin
n eine Zahl von 0 bis 4 ist;
R₁ einen oder mehrere Substituenten, die gleich oder verschieden sind, wiedergibt;
R₂ einen Kohlenwasserstoffrest wiedergibt, enthaltend 1 bis 24 Kohlenstoffatome, der einen linearen oder verzweigten, gesättigten oder ungesättigten, unsubstituierten oder substituierten, acyclischen, aliphatischen Rest; einen gesättigten oder ungesättigten, monocyclischen oder polycyclischen, unsubstituierten oder substituierten cycloaliphatischen Rest; einen gesättigten oder ungesättigten, linearen oder verzweigten, unsubstituierten oder substituierten, aliphatischen Rest, der cyclische Substituenten trägt; oder einen unsubstituierten oder substituierten aromatischen, monocyclischen oder polycyclischen Rest darstellt; oder
R₁ und R₂ zusammen einen unsubstituierten oder substituierten Cyclus, der gegebenenfalls neben dem Schwefelatom ein weiteres Heteroatom umfasst, bilden.

3. Verfahren nach Anspruch 1, wobei die aromatische Thioether-Verbindung Thioanisol, Ethyl-phenyl-sulfid, Diphenylsulfid, Phenyl-n-propyl-sulfid, Benzyl-phenyl-sulfid, Phenyl-β-phenyl-ethyl-sulfid, Phenyl-3-phenyl-propylsulfid, Allyl-phenyl-sulfid, Phenyl-propargyl-sulfid, Methallyl-phenyl-sulfid, Bis (phenylthio)-methan, 1,3-Bis(phenylthio)-propan, 1,2-Bis(phenylthio)-ethan, 1,1,3-Tris-(phenylthio)-1-propen, Chlormethyl-phenyl-sulfid, 2-Chlorethyl-phenyl-sulfid, 3-Chlorpropyl-phenyl-sulfid, 2-Bromethyl-phenyl-sulfid, Brommethyl-phenyl-sulfid, 4-Fluorbenzyl-phenyl-sulfid, 1-Chlor-4-phenyl-sulfanyl-benzol, 2-(Phenylthio)-ethanol, Methoxy-methyl-phenyl-sulfid, 2-Methoxy-ethyl-phenyl-sulfid, Azido-methyl-phenyl-sulfid, Phenyl-thio-acetonitril, 1-[(Phenyl-thio)-methyl]-piperidin, 4-[(Phenyl-sulfanyl)-methyl]-anilin, (Phenylthio)-essigsäure, 3-(Phenyl-sulfanyl)-propansäure, 4-(Phenyl-sulfanyl)-butansäure, 2-Phenyl-mercapto-methylbenzoesäure, (Phenylthio)-essigsäure-methylester, (Phenylthio)-essigsäure-ethylester, (Phenyl-thiomethyl)-trimethyl-silan, 2-[(Phenylthio)-methyl]-2-cyclopenten-1-on, (Phenylthio)-propanon, Phenyl-phenacylsulfid, Diethylphenylthio-methylphosphonat, Phenylthio-methyl, α-(Phenylthio)-acetamid, 3-(Phenylthio)-propionamid, 2-(Phenylthio)-ethanthioamid darstellt.

4. Verfahren nach Anspruch 1, wobei das Acylierungsmittel die Formel (II) 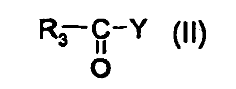 aufweist,
worin R₃ einen linearen oder verzweigten, gesättigten oder ungesättigten, unsubstituierten oder substituierten aliphatischen Rest, der 1 bis 24 Kohlenstoffatome enthält; einen monocyclischen oder polycyclischen, gesättigten oder ungesättigten, unsubstituierten oder substituierten cycloaliphatischen Rest, der 3 bis 8 Kohlenstoffatome enthält; oder einen linearen oder verzweigten, gesättigten oder ungesättigten aliphatischen Rest, der einen cyclischen Substituenten trägt; oder einen aromatischen monocyclischen oder polycyclischen, unsubstituierten oder substituierten Rest, der 3 bis 10 Kohlenstoffatome enthält, wiedergibt;
Y ein Halogenatom, eine Hydroxylgruppe, eine Gruppe -O-COR₄ oder -O-SO₂-R₄ wiedergibt;
R₄ eine der für R₃ angegebenen Bedeutungen aufweist oder C₁-C₂₄-Alkyl darstellt, das durch eine oder mehrere O unterbrochen ist und das gegebenenfalls mit Halogen substituiert ist; oder
R₃ und R₄ zusammen einen zweiwertigen linearen oder verzweigten, gesättigten oder ungesättigten aliphatischen Rest, der mindestens zwei Kohlenstoffatome enthält, bilden.

5. Verfahren nach Anspruch 1, wobei das Acylierungsmittel aus der Gruppe, bestehend aus Essigsäureanhydrid, Propansäureanhydrid, Buttersäureanhydrid, i-Buttersäureanhydrid, Trifluoressigsäureanhydrid, Benzoesäureanhydrid, Monochloressigsäureanhydrid, Dichloressigsäureanhydrid, ausgewählt ist.

6. Verfahren nach Anspruch 1, wobei der Katalysator eine Heteropolysäure oder einen Heteropolysäure enthaltenden festen Träger oder ein Salz der Heteropolysäure oder ein Gemisch davon darstellt, wobei die Heteropolysäure ein Zentralatom, umfassend P, Si, B, Ge, As, und ein koordinierendes Atom, umfassend Mo oder W oder ein Gemisch davon, aufweist.

7. Verfahren nach Anspruch 1, wobei die Heteropolysäure durch Siliziumdioxid bzw. Silica, mesoporöse Molekularsiebe, Kohlenstoff, Anionen-Tone vom Hydrotalcit-Typ oder Aluminiumoxide getragen wird.

8. Verfahren nach Anspruch 1, wobei das Salz der Heteropolysäure Li, Na, K, Rb, Cs, Mg, Ca, Fe, La, Ce, Cu, Zn, Al, Ammonium oder Gemische davon umfasst.

9. Verfahren nach Anspruch 1, wobei die Reaktionstemperatur im Bereich von Raumtemperatur bis 140°C, vorzugsweise von 40°C bis 80°C, liegt.

10. Verfahren nach Anspruch 1, wobei die Menge an Heteropolysäure oder Heteropolysäure enthaltendem festem Träger oder Salz der Heteropolysäure oder Gemisch davon zwischen 50 Gew.-% bis 0,01 Gew.-%, vorzugsweise zwischen 10 Gew.-% bis 0,1 Gew.-%, liegt.

11. Verfahren nach Anspruch 1, wobei ein molarer Überschuss des aromatischen Thioethers angewendet wird.

12. Verfahren nach Anspruch 1, wobei ein molarer Überschuss des Acylierungsmittels angewendet wird.

13. Verfahren nach Anspruch 1, wobei das Molverhältnis von dem aromatischen Thioether zu dem Acylierungsmittel 1 : 10 ist.

14. Verfahren nach Anspruch 1, wobei das Molverhältnis von dem Acylierungsmittel zu dem aromatischen Thioether 1 : 10 ist.

15. Verfahren zum Synthetisieren von aromatischen Thioetherketonen durch Umsetzen eines aromatischen Thioethers der Formel (I) nach Anspruch 2 mit einem Acylierungsmittel der Formel (II) nach Anspruch 4.

16. Verfahren nach einem der Ansprüche 1 bis 15, wobei der aromatische Thioether Thioanisol ist.

17. Verfahren nach einem der Ansprüche 1 bis 16, wobei das Acylierungsmittel i-Buttersäureanhydrid ist.

18. Verfahren nach einem der Ansprüche 1 bis 17, wobei die Heteropolysäuren H₄Si(W₃O₁₀)₄ oder H₃P(W₃O₁₀)₄ sind.

19. Verfahren nach einem der Ansprüche 1 bis 18 zum Synthetisieren von 2-Methyl-1-[4-(methylthio)-phenyl]-1-propanon durch Umsetzen von Thioanisol mit i-Buttersäureanhydrid in Gegenwart von Heteropolysäuren oder Heteropolysäure enthaltenden festen Trägern oder den entsprechenden Salzen der Heteropolysäuren.

## Revendications

1. Procédé pour synthétiser les thioéthercétones aromatiques, qui comprend la réaction d'un tioéther aromatique avec un agent d'acylation, choisi dans le groupe constitué par les acides carboxyliques, les halogénures ou les anhydrides, en présence d'hétéropolyacides ou de supports solides contenant des hétéropolyacides ou des sels d'hétéropolyacides.

2. Procédé selon la revendication 1, dans lequel le thioéther aromatique est de formule (I) 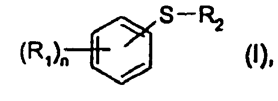 dans laquelle
n est un nombre de 0 à 4 ;
R₁ représente un ou plusieurs substituants qui sont identiques ou différents ;
R₂ représente un radical hydrocarbure contenant 1 à 24 atomes de carbone, qui est un radical aliphatique acyclique non substitué ou substitué, saturé ou insaturé, linéaire ou ramifié ; un radical cycloaliphatique non substitué ou substitué, monocyclique ou polycyclique, saturé ou insaturé ; des substituants cycliques portant un radical aliphatique non substitué ou substitué, linéaire ou ramifié, saturé ou insaturé ; ou un radical monocyclique ou polycyclique aromatique non substitué ou substitué ; ou
R₁ et R₂ forment ensemble un cycle non substitué ou substitué qui comprend éventuellement, en plus de l'atome de soufre, un autre hétéroatome.

3. Procédé selon la revendication 1, dans lequel le composé thioéther aromatique est le thioanisole, l'éthyl-phényl-sulfure, le diphényl-sulfure, le phényl-n-propyl-sulfure, benzyl-phényl-sulfure, phényl-β-phényléthyl-sulfure, phényl-3-phénylpropyl-sulfure, allyl-phényl-sulfure, phényl-propargyl-sulfure, méthallylphényl-sulfure, bis(phénylthio)méthane, 1,3-bis(phénylthio)propane, 1,2-bis(phénylthio)éthane, 1,1,3-tris(phénylthio)-1-propène, chorométhyl-phényl-sulfure, 2-chloroéthyl-phényl-sulfure, 3-chloropropyl-phényl-sulfure, 2-bromoéthyl-phényl-sulfure, bromométhyl-phényl-sulfure, 4-fluorobenzyl-phényl-sulfure, 1-chloro-4-phénylsulfanyl-benzène, 2-(phénylthio)éthanol, méthoxyméthyl-phényl-sulfure, 2-méthoxyéthyl-phényl-sulfure, azidométhyl-phényl-sulfure, phénylthioacétonitrile, 1-[(phénylthio)méthyl]pipéridine, 4-[(phénylsulfanyl)méthyl]aniline, acide (phénylthio)acétique, acide 3-(phénylsulfanyl)propanoïque, acide 4-(phénylsulfanyl)-butanoïque, acide 2-phénylmercaptométhylbenzoïque, méthyl(phénylthio)acétate, éthyl(phénylthio)acétate, (phénylthiométhyl)triméthylsilane, 2-[(phénylthio)méthyl]-2-cyclopentén-1-one, (phénylthio)propanone, phényl-phénacyl-sulfure, diéthyl phénylthiométhylphosphonate, phénylthiométhyle, α-(phénylthio)acétamide, 3-(phénylthio)propionamide, 2-(phénylthio)éthanethioamide.

4. Procédé selon la revendication 1, dans lequel l'agent d'acylation est de formule (II) : 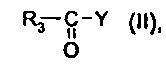 dans laquelle
R₃ représente un radical aliphatique non substitué ou substitué, saturé ou insaturé, linéaire ou ramifié, contenant 1 à 24 atomes de carbone ; un radical cycloaliphatique non substitué ou substitué, saturé ou insaturé, monocyclique ou polycyclique contenant 3 à 8 atomes de carbone ; ou un radical aliphatique saturé ou insaturé, linéaire ou ramifié, portant un substituant cyclique ; ou un radical non substitué ou substitué monocyclique ou polycyclique aromatique contenant 3 à 10 atomes de carbone ;
Y représente un atome d'halogène, un groupe hydroxyle, un groupe -O-COR₄ ou -O-SO₂-R₄ ;
R₄ a l'une des significations données pour R₃, ou est un alkyle en C₁-C₂₄ qui est interrompu par un ou plusieurs O et qui est éventuellement substitué par un halogène : ou
R₃ et R₄ forment ensemble un radical aliplaatique saturé ou insaturé, linéaire ou ramifié, divalent, contenant au moins 2 atomes de carbone.

5. Procédé selon la revendication 1, dans lequel l'agent d'acylation est choisi dans le groupe constitué par l'anhydride acétique, l'anhydride propanoïque, l'anhydride butyrique, l'anhydride i-butyrique, l'anhydride trifluoroacétique, l'anhydride benzoïque, l'anhydride monochloroacétique, l'anhydride dichloroacétique.

6. Procédé selon la revendication 1, dans lequel le catalyseur est un hétéropolyacide ou un support solide contenant un hétéropolyacide ou un sel de l'hétéropolyacide ou un mélange de ceux-ci, dans lequel l'hétéropolyacide possède un atome central comprenant P, Si, B, Ge, As et un atome de coordination comprenant Mo ou W ou un mélange de ceux-ci.

7. Procédé selon la revendication 1, dans lequel l'hétéropolyacide est supporté sur de la silice, des tamis moléculaires mésoporeux, du carbone, des argiles anioniques du type hydrotalcite ou des oxydes d'aluminium.

8. Procédé selon la revendication 1, dans lequel le sel de l'hétéropolyacide comprend Li, Na, K, Rb, Cs, Mg, Ca, Fe, La, Ce, Cu, Zn, Al, de l'ammonium ou des mélanges de ceux-ci.

9. Procédé selon la revendication 1, dans lequel la température de réaction va de la température ambiante à 140°C, de préférence de 40°C à 80°C.

10. Procédé selon la revendication 1, dans lequel la quantité d'hétéropolyacide ou de support solide contenant un hétéropolyacide ou de sel de l'hétéropolyacide ou du mélange de ceux-ci est compris entre 50 % en poids à 0,01 % en poids, de préférence entre 10 % en poids et 0,1 % en poids.

11. Procédé selon la revendication 1, dans lequel un excès molaire du thioéther aromatique est utilisé.

12. Procédé selon la revendication 1, dans lequel un excès molaire de l'agent d'acylation est utilisé.

13. Procédé selon la revendication 1, dans lequel le rapport molaire du thioéther aromatique à l'agent d'acylation va de 1 à 10.

14. Procédé selon la revendication 1, dans lequel le rapport molaire de l'agent d'acylation au thioéther aromatique va de 1 à 10.

15. Procédé pour synthétiser les thioéthercétones aromatiques en faisant réagir un tioéther aromatique de formule (I) selon la revendication 2 avec un agent d'acylation de formule (II) selon la revendication 4.

16. Procédé selon l'une quelconque des revendications 1 à 15, dans lequel le thioéther aromatique est le thioanisole.

17. Procédé selon l'une quelconque des revendications 1 à 16, dans lequel l'agent d'acylation est l'anhydride i-butyrique.

18. Procédé selon l'une quelconque des revendications 1 à 17, dans lequel les hétéropolyacides sont H₄Si(W₃O₁₀)₄ ou H₃P(W₃O₁₀)₄.

19. Procédé selon l'une quelconque des revendications 1 à 18 pour synthétiser la 2-méthyl-1-[4-(méthylthio)phényl]-1-propanone en faisant réagir le thioanisole avec l'anhydride i-butyrique en présence d'hétéropolyacides ou de supports solides contenant un hétéropolyacide ou de sels d'hétéropolyacides correspondants.
